(19) Europäisches Patentamt
European Patent Office
Office européen des brevets

(11) Veröffentlichungsnummer: **0 328 997 A2**

(12) # EUROPÄISCHE PATENTANMELDUNG

(21) Anmeldenummer: **89102086.9**

(22) Anmeldetag: **08.02.89**

(51) Int. Cl.4: **A61K 45/06 , A61K 31/56 , //(A61K45/06,31:56,31:557, 31:20),(A61K31/56,31:557, 31:20)**

Patentansprüche für folgende Vertragsstaaten: ES + GR.

(30) Priorität: **18.02.88 DE 3805064**

(43) Veröffentlichungstag der Anmeldung: **23.08.89 Patentblatt 89/34**

(84) Benannte Vertragsstaaten: **AT BE CH DE ES FR GB GR IT LI LU NL SE**

(71) Anmelder: **HOECHST AKTIENGESELLSCHAFT
Postfach 80 03 20
D-6230 Frankfurt am Main 80(DE)**

(72) Erfinder: **Weithmann, Klaus Ulrich, Dr.
Am Domherrnwald 18
D-6238 Hofheim am Taunus(DE)**

(54) **Pharmazeutische Zubereitungen zur Weheninduktion und zur Verwendung als Kontrazeptivum.**

(57) Die vorliegende Erfindung betrifft ein pharmazeutisches Kombinationspräparat zur Weheninduktion bzw. zur Verhinderung des Eintretens einer Schwangerschaft, enthaltend

A) ein oder mehrere Antigestagen(e)

B) eine oder mehrere langkettige polyungesättigte Fettsäure(n) bzw. deren Zubereitungen und, falls erforderlich

C) Prostaglandine,

wobei als Antigestagene alle Verbindungen in Frage kommen, die eine starke Affinität zum Gestagenrezeptor besitzen, dabei aber selbst keine nennenswerte eigene gestagene Aktivität zeigen.

Die Präparate werden bevorzugt intravaginal oder (extra)-amnial appliziert. Dabei können die Antigestagene und die erfindungsgemäßen Fettsäuren entweder gleichzeitig oder getrennt, z.B. nacheinander verabreicht werden.

EP 0 328 997 A2

## Pharmazeutische Zubereitungen zur Weheninduktion und zur Verwendung als Kontrazeptivum

Pharmakologische und chirurgische Methoden, mit deren Hilfe an Säugetieren und in der Humanmedizin ein vorzeitiger Schwangerschaftsabbruch bzw. die künstliche Einleitung der Geburt durchgeführt werden kann, sind bereits bekannt.

In EP-A-139608 ist der Stand der Technik hinsichtlich pharmakologischer Methoden beschrieben. So kann ein Schwangerschaftsabbruch z.B. durch die medizinische Applikation von bestimmten Prostaglandinen oder, alternativ, durch Gabe von Antigestagenen (Antiprogesteronen, Progesteronantagonisten, Antiprogestomimetika) ausgelöst werden. Nachteilig bei beiden Methoden ist ihre nicht hundertprozentige Zuverlässigkeit. Darüber hinaus führt insbesondere die Anwendung von Prostaglandinen häufig zu unerwünschten medizinischen Nebenwirkungen. Es wird daher in EP-A-139608 (vgl. auch EP-A-0184471) vorgeschlagen, mit Hilfe der kombinierten Gabe von Prostaglandinen und Antigestagenen die Zuverlässigkeit der Methode zu erhöhen, bei gleichzeitig verringerter Nebenwirkungsrate.

Es ist nun allerdings bekannt, daß Prostaglandine nicht nur uteroaktive Wirkungen haben, sondern daß sie darüber hinaus an zahlreichen biologischen Prozessen beteiligt sind. In der Literatur ist beschrieben, daß Prostaglandine in den verschiedensten Zelltypen synthetisiert werden. Sie wirken dort als essentielle Transmitter- bzw. Modulatorsubstanzen bei der biochemischen Signalübertragung, und sind dabei an vielfältigen biologischen Reaktionen, wie z.B. der Schmerz- und Fieberexpression, der Beeinflussung des Muskeltonus, der dilatatorischen bzw. konstriktorischen, Gefäßmuskelspannung sowie der Thromboseneigung des Blutes beteiligt. Wegen dieser vielfältig aufgefächerten biologischen Wirkungen der Prostaglandine ist es daher verständlich, daß ihre externe medizinische Anwendung, erfolge sie nun systemisch oder lokal, nicht nur die erwünschten therapeutischen Effekte in der Zielzelle hevorrufen wird, sondern zahlreiche biologische Nebeneffekte, die nicht zur medizinisch erwünschten Wirkung beitragen, und die häufig sogar schädlich sind. Die medizinische Applikation von Prostaglandinen ist folglich gemäß dem Stand der Technik, auch bei reduzierten Dosierungen, als problematisch hinsichtlich der unerwünschten Nebenwirkungen anzusehen.

Gemäß der vorliegenden Erfindung wurde nun gefunden, daß die prostaglandintypischen Nebenwirkungen der o.g. Prostaglandin/Antiprogesteron-Kombinationen überraschenderweise vermieden werden können, wenn der Prostaglandinanteil vollständig oder teilweise durch langkettige, mehrfache ungesättigte Fettsäuren bzw. durch geeignete Zubereitungen solcher Fettsäuren (s. EP-A-244832) ersetzt wird. Von besonderer Bedeutung ist es hierbei, daß die Anwendung dieser Fettsäuren, bzw. der Fettsäurenzubereitung, in der Regel lokal (topisch), insbesondere amnial oder intravaginal, gegebenenfalls in Form der in der Galenik bekannten transdermalen Systeme, erfolgt.

Bei Anwendung derartiger Kombinationen aus Antiprogesteron und den genannten Fettsäuren (bzw. Zubereitungen) treten die o.g. unerwünschten, insbesondere Prostaglandin- typischen Nebenwirkungen nicht auf, obwohl die Zuverlässigkeit der erwünschten Wirkung erfindungsgemäß besser ist, als man aufgrund der additiven Wirkung der Einzelkomponenten erwarten würde. Es sei hier angemerkt, daß unter "erwünschter Wirkung" nicht nur die Geburtseinleitung und die Induktion des Schwangerschaftsabbruchs, sondern erfindungsgemäß auch die Inhibition der Nidation des Ovulums zu verstehen ist.

Gegenstand der vorliegenden Erfindung sind daher pharmazeutische Kombinationserzeugnisse zur gemeinsamen Verwendung für die Einleitung der Geburt, für den Schwangerschaftsabbruch bzw. zur Verhinderung des Eintretens einer Schwangerschaft, enthaltend

A) ein oder mehrere Antigestagen(e) und

B) eine oder mehrere langkettige polyungesättigte Fettsäure(n) bzw. Zubereitungen derselben, und falls erforderlich,

C) Prostaglandine (insbesondere die in EP-A-139608, Seite 3-4 genannten), wobei

als A) Antigestagene alle Verbindungen in Frage kommen, die eine starke Affinität zum Gestagenrezeptor (Progesteronrezeptor) besitzen, dabei aber keine nennenswerte eigene gestagene Aktivität zeigen. Als Progesteronantagonisten kommen beispielsweise folgende Steroide in Frage:

11ß[(4-N,N-Dimethylamino)-phenyl]17β-hydroxy-17-propinyl-4,9(10)-estradien-3-on (RU 38 486; s.EP-A-139 608) 11ß- [(4-N,N-Dimethylamino)-phenyl]-17β-hydroxy-18-methyl-17α-propinyl-4,9(10)-estradien-3-on und 11ß-[(4-N,N-Dimethylamino)-phenyl]-17aß-hydroxy-17aα -propinyl-D-homo-4,9(10)-16-estratrien-3-on, 17β-hydroxy-11ß-[4-N,N-Dimethylaminophenyl]-17α-[3-hydroxy-1-propenyl]estra-4,9-dien-3-on,

ferner

11ß-p-Methoxyphenyl-17βhydroxy-17α-ethinyl-4,9(10)-estradien-3-on (Steroids 37 (1981) 361-382) und 11ß- (4-Dimethylaminophenyl)-17α-hydroxy-17β(3-hydroxypropyl)-13α-methyl-4,9-gonadien-3-on (s.EP-A-139608) sowie auch die in EP-A-184471 beschriebenen Antigestagene und als B) langkettige polyungesättigte Fettsäuren bzw. deren pharmazeutische Zubereitungen, wie eine oder mehrere ungesättigte Fettsäure-(n); gekennzeichnet durch zwei, drei, vier oder fünf isoliert angeordnete Doppelbindungen und 18 bis 22 geradkettig angeordnete Kohlenstoffatome, die an einem oder an zwei C-Atomen der Position 2, 3, 4, 16, 17, 18, 19, 20 methyliert oder äthyliert sein können, als freie Carbonsäure mit endständigem $-CO_2H$ oder $-CO_2X$, wobei X eine unter sauren Bedingungen abspaltbare Schutzgruppe, wie ein Alkylrest, z.B. ein Methyl-, Äthylrest oder ein Metall- oder Amin-Kation oder die kationische Form eines Ionenaustauschers darstellt, oder als Carbonsäureamid gegebenenfalls kombiniert mit Verbindungen, wie

$B_1$) eine phenolische Verbindung der Formel I

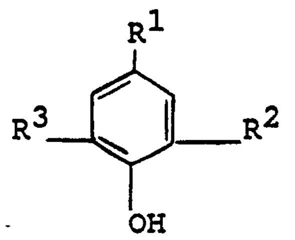

I

in der die Reste $R^2$ und $R^3$ Hydroxylgruppen, oder Wasserstoff und $R^1$ den Rest -OH, $CO_2H$, $-CH_2-CO_2H$, $-CH=CH-CO_2H$, $-CH_2-CHR^4-R^5$, $-CHOH-CH_2-NH-R^6$ mit $R^4$ = -H oder $-CO_2H$ und $R^5$ = -H oder $-NH_2$ und $R^6$ = -H, $CH_3$ oder $-C_2H_5$ bedeuten,

$B_2$) ein Indolderivat der Formel II

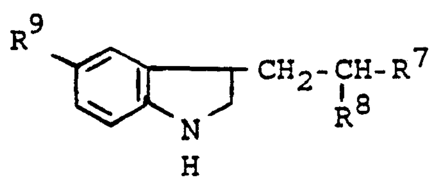

II

in der $R^7$ Wasserstoff oder COOH, $R^8$ Wasserstoff oder $-NH_2$ und $R^9$ Wasserstoff oder -OH bedeuten,

$B_3$) Cystein oder Homocystein, oder Liponsäure, deren acyclischer Alkylrest um bis zu vier Methylengruppen verkürzt sein kann,

$B_4$) ein Peptid, bestehend aus maximal zehn Aminosäuren, bei dem eine oder mehrere der Aminosäuren durch jeweils eine der Verbindungen gemäß $B_1$) bis $B_3$) ersetzt ist,

$B_5$) eine Verbindung gemäß $B_1$) bis$B_4$), die an einem N-Atom eine $C_1$-$C_4$-Alkanoylgruppe tragen kann,

$B_6$) einen Flavonabkömmling, der mit wenigstens einer Hydroxylgruppe, die einen Zuckerrest tragen kann, substituiert ist,

$B_7$) ein Salz der ionischen Formen der Verbindungen gemäß $B_1$)-$B_6$) und gegebenenfalls

$B_8$) eine Carbonsäureverbindung gemäß $B_1$) bis $B_7$), die mit einem Alkoxy-Rest verestert sein kann oder als Carbonsäureamid vorliegt, das auch mono- oder dialkyliert sein kann; und gegebenenfalls als Stabilisatoren eine oder mehrere Verbindungen der folgenden Gruppen:

$B_9$) Dimethylsulfoxid, Äthylalkohol, Glycerin, Äthylenglykol, Polyäthylenglykol oder Glycerintriacetat,

$B_{10}$) Phospholipide, Zuckerlipide, Cyclodextrine, Proteine, z.B. solche aus Human- und Säugetierblut herstellbare, oder Vitamine der E-Reihe.

Als Metallkationen X können z.B. solche der Alkalimetalle wie Lithium, Natrium und Kalium, und der Erdalkalimetalle wie Magnesium und Calcium, aber auch kationische Formen anderer Metalle, wie Aluminium, Zink und Eisen verwendet werden, gegebenenfalls chelatisiert mit Zitronensäure oder Äthylendiamintetraessigsäure und dgl. Aminkationen sind solche von primären, sekundären oder tertiären Aminen wie der Alkylamine, z.B. Mono-, Di, und Trimethyl- bzw. -äthyl-, -propyl-, -isopropyl-, -butyl-, -isobutyl-, -t-butylamin, sowie N(Methylhexyl)-, N-Methyl-hexyl-, Benzyl-ß-phenyl-äthylamin, Äthylendiamin, Diäthylentriamin, Pyrrolidin, Piperidin, Morpholin, Piperazin, Mono-, Di- und Triäthanolamin, Äthyldiäthanolamin, N-Butyläthanolamin, Tris-(hydroxymethyl)-aminomethan, und dergleichen. Geeignete Aminsalze sind z. B. die des Tryptamins, Cysteins sowie die basischen Aminsalze des Lysins und des Arginins. Geeignete quaternäre Ammoniumkationen sind z. B. das Tetramethylammonium und das Benzyltrimethylammonium. Diese Kationen können auch zur Salzbildung der anionischen Formen der Verbindungen gemäß $B_1$) bis $B_6$)

verwendet werden, wohingegen zur Salzbildung bei den kationischen Formen Chlorid und Fluorid bevorzugt sind.

Bevorzugt eingesetzt werden die Fettsäuren mit den Bezeichnungen 18:2ω-6, 20:4ω-6, 22:5ω-6, 18:3ω-3, 20:5ω-3, 22:6ω-3, 18:3ω-6, 20:3ω-6, 22:3ω-6, 22:4ω-6 und 22:4ω-3, wobei in der üblichen Nomenklatur die erste Zahl die Anzahl der Kohlenstoffatome, die Zahl nach dem Doppelpunkt die Anzahl der Doppelbindungen und die Zahl nach dem ω die Positionen der ersten Doppelbindung gerechnet vom Methylende des Moleküls bedeutet. Besonders bevorzugt sind Arachidonsäure (20:4ω-6) und Dihomogammalinolensäure (20:3ω-6).

Die für die genannten biologischen Anwendungen erforderlichen Anti-Gestagen-Dosierungen in der erfindungsgemäßen Kombinationstherapie liegen in der Regel unterhalb der bei einer Antigestagen-Monotherapie erforderlichen Dosierungen. Im allgemeinen genügen als Tagesdosis bei Humananwendungen z.B. 10-400 mg 11ß [(4-N,N-Dimethylamino)-phenyl]-17ß-hydroxy-17α-propinyl-4,9 (10)-estradien-3-on (RU 38486, identisch mit RU 486). Selbstverständlich kann RU 38486 durch biologisch äquivalente Mengen anderer Antigestagene ersetzt werden. Im einzelnen hängt die erforderliche Dosierung einerseits von der biologischen Reaktion des zu behandelnden Menschen oder Tieres ab, die wiederum vom Alter, Zustand etc. abhängig ist, und andererseits von der Art der Applikation, die oral, subcutan enteral oder parenteral mit Hilfe von Pillen, Kapseln, Dragees, Tabletten, Lösungen oder Suspensionen usw. erfolgen kann. Bevorzugt ist die (topische bzw. sub-oder transcutane) intravaginale oder (extra-)-amniale Applikation, die beispielsweise mittels Vaginalzäpfchen oder transdermaler Systeme wie Hautpflaster durchgeführt werden kann. Die galenischen Formen enthalten in der Regel Zusätze und Trägersubstanzen gemäß dem Stand der Technik, damit eine gute Hautpenetration gewährleistet ist. Bezogen auf RU 38486 enthält eine Dosiseinheit 10-200 vorzugsweise 50 -150 mg, für andere Antigestagene wird sie gemäß deren biologischen Äquivalenzen berechnet.

Die Applikation der langkettigen, polyungesättigten Fettsäuren erfolgt erfindungsgemäß (topisch, sub- oder transcutan) intravaginal, z.B. durch Vaginalzäpfchen oder (extra-)amnial, gegebenenfalls durch transdermale Systeme und, falls erforderlich, mit Zusätzen und Trägersubstanzen gemäß dem technischen Stand der Galenik. Die Tagesdosis, bezogen auf Arachidonsäure oder Dihomogammalinolensäure, liegt zwischen 1 und 2000 mg, im allgemeinen zwischen 2 und 1000 mg und vorzugsweise zwischen 5 und 500 mg. Die Dosiseinheit enthält 1 bis 1000, vorzugsweise 5 bis 500 mg Arachidonsäure (Dihomogammalinolensäure). Die für andere erfindungsgemäße Fettsäuren erforderlichen Dosierungen können mit dem Maß ihrer biologischen Wirkung als Cyclooxygenase-Substrat leicht berechnet werden. Besonders vorteilhaft ist es, die oben beschriebenen Fettsäurenzubereitungen, enthaltend eine oder mehrere der Verbindungen $B_1$-$B_{10}$, bevorzugt in den in EP-A-244 832 genannten Dosisverhältnissen anzuwenden.

Die Dosierungen aller genannten Wirkstoffe sind bei der Anwendung an Tieren, insbesondere entsprechend deren Gewicht und deren biologischen Reaktionen, zu erniedrigen oder zu erhöhen.

Die erfindungsgemäße Kombinationsapplikation erfolgt häufig über 1 bis 3 oder 4, vorzugsweise 1 bis 2 Tage und kann gegebenenfalls nach 5 bis 20 Tagen wiederholt werden. Dabei können die Antigestagene und die erfindungsgemäßen Fettsäuren sowohl getrennt als auch bevorzugt gleichzeitig, aber auch nacheinander (sequentiell) in einem Gewichtsverhältnis von Antigestagen zu Fettsäuren wie 1:1 bis 1:300 angewendet werden. Ein Gewichtsverhältnis von <1:20, insbesondere <1:30, ist bevorzugt.

Für die lokale (topische) intravaginale bzw. (extra-)amniale Applikation ist es vorteilhaft, die erfindungsgemäße Fettsäure und das Antigestagen in einer Dosiseinheit, z.B. einem Zäpfchen (Suppositorium) zusammenzufassen. Falls erforderlich, kann durch Zusatz von geeigneten Retardierungsmitteln (z.B. Eudragit®) wahlweise die Freisetzung der erfindungsgemäßen Fettsäuren oder des Antigestagens aus der galenischen Form verzögert werden. Die erfindungsgemäßen Fettsäuren bzw. deren Zubereitungen, können auch in getrennten galenischen Einheiten enthalten sein, die aber in einer Arzneimittelpackung zur gemeinsamen Verwendung zusammengefaßt sind.

## Beispiele

### Beispiel 1:

50 mg 11ß-[4-N,N-Dimehylamino)-phenyl]-17ß-hydroxy-17α-propinyl-4,9(10)-estradien-3-on werden in 30 ml 96 % Äthanol gelöst. Die Lösung wird wie in "Fertility and Sterility" 44, (1985) Seite 263 beschrieben, auf ein handelsübliches Tampon aufgetragen und bei 40 °C getrocknet. Hierauf werden in

analoger Weise 50 ml einer ebenfalls 96 %igen Äthanollösung, die 50 mg Dihomogammalinolensäure und 100 mg Liponsäure enthält, aufgetragen und bei einer Maximaltemperatur von 30 °C getrocknet.

**Beispiel 2:**

Ein die Wirkstoffe enthaltendes Suppositorium wird hergestellt, indem 8 g Kakaofett in einer geeigneten Form mit Hilfe einer IR-Lampe geschmolzen (Maximaltemperatur = 34 °C) und die Schmelze mit 50 mg Arachidonsäure sowie 5 mg des genannten Antiprogesterons vermengt wird. Nach Hinzufügen von etwas (ca. 20 mg) geraspeltem, festem Kakaofett wird die Schmelze im Kühlschrank abgekühlt. Bei der Lagerung der Suppositorien ist jeglicher Einfluß von Feuchtigkeit, Wärme und Licht zu vermeiden.

**Beispiel 3:**

10 g einer wässrigen Lösung, die 15 Gewichtsprozent Gelatine und 0,5 Gewichtsprozent Glycerin enthält, wird vorsichtig in einer Schmelzform erwärmt und die gebildete homogene Masse unter gelinden Erwärmen mit 20 mg des o.g. Antiprogesterons, 30 mg Dihomogammalinolensäure, sowie, bei Bedarf mit 30 mg N-Acetylcysteamin, bis zur Homogenität vermischt und schließlich abgekühlt.

**Beispiel 4:**

Eine Folie enthält: 0,72 mg Polyoxyethylenpolyoxypropylenpolymeres, (Pluronic F 68®), 41 mg Hydroxypropylcellulose, 100 mg Natriumarachidonat. Die Folie wird gleichmäßig mit einer Suspension aus 100 mg Kakaobutter und 10 mg 11$\beta$-(4,N,N-Dimethyl-amino)-phenyl-17b-hydroxy-17$\alpha$-propinyl-4,9(10)-estradien-3-on beschichtet.

**Beispiel 5:**

Bestimmung der Prostaglandin-F-2-alpha-synthese im Uterus

Weibliche Wistar-Ratten (ca. 150 g) wurden mit 30 mcg Beta-Östradioldiacetat s.c. behandelt, am nächsten Tag getötet, die Uteri entnommen und in 7,3 ml 50 mmol/l Kaliumphosphatpuffer (pH = 8) unter Zusatz von 6 mg/l C-14-Arachidonsäure homogenisiert, zentrifugiert und einmal mit Puffer gewaschen. Jeweils 250 mcl der erhaltenen Suspension wurden mit den in Tab. 1 angegebenen Wirkstoffen versetzt und dann 30 min bei 37 °C inkubiert. Nach dem Abstoppen des Reaktionsansatzes mit Hilfe von 50 mcl 1,2 mol/l Zitronensäure wurde zweimal mit jeweils 0,75 ml Essigsäureäthylester extrahiert, der Ester mit Stickstoff verblasen und der Rückstand in 0,1 ml Methanol gelöst. Die Auftrennung der gebildeten Produkte erfolgte mittels Hochdruckflüssigkeitschromatographie wie ausführlich in EP 0244832, Seite 18, beschrieben. Die Quantifizierung von Prostaglandin F-2-alpha erfolgte mit Hilfe der Peakfläche.

| Experiment Nr. | Zusatz von | rel. Menge Prostaglandin-F-2-alpha | % Zunahme gegen Kontrolle |
|---|---|---|---|
| I | ohne | 243 | 0 |
| II | 3 mg/l Progesteron | 289 | 18,9 |
| III | 3 mg/l RU 38486 | 314 | 29,6 |
| IV | 3 mg/l Progesteron plus 3 mg/l RU 38486 | 400 | 64,6 |

Durch Zusatz von Progesteron konnte die Synthese von Prostaglandin-F-2-alpha stimuliert werden (siehe Spalte % Zunahme ...), noch deutlicher war die Steigerung nach Gabe von RU 38486, sowie der Kombination aus Progesteron und RU 38486. Durch Zugabe der Thiolverbindung Glutathion konnte die Synthese von Prostaglandin-F-2-alpha darüberhinaus noch um 150 % gesteigert werden. Einer derartige Stimulierung war auch nach Erhöhung der zugesetzten Arachidonsäuremenge auf 30 mg/l meßbar. Durch Zusatz von 75 mg/l Arachidonsäure konnte die Syntheserate noch um weitere ca. 30 % gesteigert werden.

Mit höheren Arachidonsäure-Gaben war die Zunahme der Stimulation schwächer. Ein günstiges Gewichtsverhältnis von Antiprogesteron und polyungesättigter Fettsäure beträgt demnach etwa 1 : 10 bis 1 : 25. Wird das Gewichtsverhältnis kleiner als 1 : 100, dann nimmt die Synthesegeschwindigkeit für Prostaglandin-F-2-alpha nur noch langsam zu.

**Beispiel 6:**

Neben Prostaglandin-F-2-alpha wird der Uterustonus bzw. die Uteruskontraktilität auch durch den cycloAmp-Spiegel des Gewebes bestimmt, siehe Nature, Vol. 225 (17.1.1970), Seite 282.

**Bestimmung von cycloAmp im Uterus**

Wie in Beispiel 5 beschrieben, wird Uterushomogenat aus betaöstradioldiacetat-behandelten Ratten hergestellt. Der zur Homogenisierung verwendete Puffer besteht aus 0,14 mol/l NaCl, 5 mmol/l KCl, 10 mmol/l Glucose, 10 mmol/l Essigsäure, 2 mmol/l Dinatriumhydrogenphosphat, 20 mmol/l Tris-HCl, 0,001 % Phenolrot, 1,2 mol/l $MgSO_4$, 0,8 mmol/l $CaCl_2$ und 0,01 mmol/l Isobutylmethylxanthin. Durch Einleiten von $CO_2$-Gas wird dieser Puffer vor Gebrauch auf pH = 7,4 gebracht. 10 ml des Uterushomogenates werden nun mit 0,03 mmol/l tritiiertem Adenin 40 Minuten bei 37 °C inkubiert. Das Gewebe wird nun filtriert, wieder in obigem Puffer aufgenommen und anschließend in der Weise aufgeteilt, daß jede Portion in 10 ml des genannten Puffers suspendiert ist. Nach einer Vorinkubationsphase von 5 Minuten werden die in Tab. 2 genannten Wirkstoffe hinzugefügt und das Gemisch für weitere 100 Minuten inkubiert. Dann wird wieder filtriert, das Gewebe in flüssigem Stickstoff tiefgefroren, verrieben und das erhaltene Pulver in 1 ml 6 %iger Trichloressigsäure homogenisiert. CycloAMP wird abschließend durch Säulenchromatographie wie in Analytical Biochemistry 58, (1974) 541-548 (insbesondere Methode C S. 543) abgetrennt und quantifiziert. Tab. 2 zeigt die Ergebnisse:

| Experiment | Zusatz | cycloAMP (% Umsatz) | Differenz zur Kontrolle |
|---|---|---|---|
| I | ohne (Kontrolle) | 0,53 | - |
| II | 2 mmol/l Arachidonsäure | 1,06 | 0,53 |
| III | 3 mmol/l Dihomogammalinolensäure | 1,45 | 0,92 |
| IV | 0,1 mmol/l RU 38486 | 0,92 | 0,39 |

| Experiment | Zusatz | cycloAMP (% Umsatz) | Differenz zur Kontrolle |
|---|---|---|---|
| V | 0,1 mmol/l RU 38486 plus 3 mmol/l Arachidonsäure | 2,49 | 1,96 |
| VI | 0,1 mmol/l RU 38486 plus 3 mmol/l Dihomogammalinolensäure | 3,19 | 2,66 |
| VII | 0,1 mmol/l RU 38486 plus 3 mmol/l Eicosapentaensäure (20 : 5, $\omega$ - 3) | 0,95 | 0,42 |

Als Ergebnis zeigte sich insbesondere, daß die Kombination von Antiprogesteron mit Dihomogammalinolensäure noch wirksamer die Uteruskontraktilität über den cycloAmp-Spiegel beeinflussen kann als die Kombination mit Arachidonsäure (Experiment V und VI), während Eicosapentaensäure (20 : 5, $\omega$ - 3) fast unwirksam war (Experiment VII).

| | | | |
|---|---|---|---|
| VIII | 0,1 mmol/l RU 38486 plus 3 mmol/l Dihomogammalinolensäure plus 0,2 mcmol/l Prostaglandin $E_1$ | 3,52 | 2,99 |

Durch Zusatz von wenig Prostaglandin $E_1$ kann eine zusätzliche Stimulierung der Uteruskontraktilität über den cycloAmp-Spiegel erreicht werden (Experiment VIII).

## Ansprüche

1. Pharmazeutisches Kombinationspräparat, dadurch gekennzeichnet, daß es

a) mindestens ein Antigestagen, und

b) eine oder mehrere langkettige, mehrfach ungesättigte Fettsäure(n), gegebenenfalls unter Zusatz von Stabilisatoren und/oder Stimulatoren der Prostaglandinsynthese,

c) sowie gegebenenfalls mindestens ein Prostaglandin enthält.

2. Präparat nach Anspruch 1, dadurch gekennzeichnet, daß als Antigestagene Verbindungen verwendet werden, die eine starke Affinität zum Gestagenrezeptor, aber keine nennenswerte gestagene Aktivität zeigen.

3. Präparate nach Anspruch 1, dadurch gekennzeichnet, daß als Antigestagene vorzugsweise die folgenden Verbindungen verwendet werden:
11β-[(4-N,N-Dimethylamino)-phenyl]-17β-hydroxy-17α-propinyl-4,9(10)-estradien-3-on,
11β-[(4-N,N-Dimethylamino)-phenyl]-17β-hydroxy-18-methyl-17α-propinyl-4,9(10)-estradien-3-on,
11β-[(4-N,N.Dimethylamino)-phenyl]-17aβ-hydroxy-17aα-propinyl-D-homo-4,9(10)-16-estratrien-3-on,
11β-p-Methoxyphenyl-17β-hydroxy-17α-ethinyl-4,9(10)-estradien-3-on oder
11β-(4-Dimethylaminophenyl)-17β-hydroxy-17β-(3-hydroxypropyl)-13α-methyl-4,9-gonadien-3-on

4. Präparat nach Anspruch 1, dadurch gekennzeichnet, daß als langkettige polyungesättigte Fettsäuren solche verwendet werden, die 3 bis 5 isoliert angeordnete Doppelbindungen und 18 bis 22 geradkettig angeordnete C-Atome, welche an einem oder 2 C-Atomen der Position 2, 3, 4, 16, 17, 18, 19 oder 20 methyliert oder äthyliert sein können, enthalten, und die Endgruppe $-CO_2X$ besitzen, wobei X Wasserstoff, die Aminogruppe, eine unter sauren Bedingungen abspaltbare Schutzgruppe, ein Metall- oder Aminokation oder die kationische Form eines Ionenaustauschers darstellt.

5. Präparat nach Anspruch 1, dadurch gekennzeichnet, daß die Fettsäuren 18:2ω-6, 20:4ω-6, 22:5ω-6, 18:3ω-3, 20:5ω-3, 22:6ω-3, 18:3ω-6, 20:3ω-6, 22:3ω-6, 22:4ω-6, 22:4ω-3 verwendet werden.

6. Präparat nach Anspruch 1, dadurch gekennzeichnet, daß die Fettsäuren 20:4ω-6 und/oder 20:3ω-6 verwendet werden.

7. Präparat nach Anspruch 1, dadurch gekennzeichnet, daß gegebenenfalls Prostaglandine der E und/oder F-Reihe verwendet werden.

8. Präparat nach Anspruch 1, dadurch gekennzeichnet, daß es Antigestagene und polyungesättigte Fettsäuren in einem Gewichtsverhältnis von 1:1 bis 1:300 enthält.

9. Präparat nach Anspruch 1, dadurch gekennzeichnet, daß es Antigestagene und polyungesättigte Fettsäuren in einem Gewichtsverhältnis von <1 : 20 enthält.

10. Präparat nach Anspruch 1, dadurch gekennzeichnet, daß es pro Dosierungseinheit 1 - 500 mg einer polyungesättigten Fettsäure enthält.

11. Verwendung von Antigestagenen und langkettigen polyungesättigten Fettsäuren gemäß Anspruch 1, zur gemeinsamen oder gleichzeitigen getrennten oder zeitlich abgestuften Anwendung zur Geburtseinleitung, für den Schwangerschaftsabbruch, oder als Kontrazeptivum.

Patentansprüche für folgende Vertragsstaaten: ES, GR

1. Verfahren zur Herstellung von pharmazeutischen Präparaten, dadurch gekennzeichnet, daß man

a) mindestens ein Antigestagen, und

b) eine oder mehrere langkettige, mehrfach ungesättigte Fettsäure(n), gegebenenfalls unter Zusatz von Stabilisatoren und/oder Stimulatoren der Prostaglandinsynthese,

c) sowie gegebenenfalls mindestens ein Prostaglandin kombiniert.

2. Verfahren nach Anspruch 1, dadurch gekennzeichnet, daß als Antigestagene Verbindungen verwendet werden, die eine starke Affinität zum Gestagenrezeptor, aber keine nennenswerte gestagene Aktivität zeigen.

3. Verfahren nach Anspruch 1, dadurch gekennzeichnet, daß als Antigestagene vorzugsweise die folgenden Verbindungen verwendet werden.
11β-[(4-N,N-Dimethylamino)-phenyl]-17β-hydroxy-17α-propinyl-4,9(10)-estradien-3-on,
11β-[(4-N,N-Dimethylamino)-phenyl]-17β-hydroxy-18-methyl-17α-propinyl-4,9(10)-estradien-3-on,

11$\beta$-[(4-N,N.Dimethylamino)-phenyl]-17a$\beta$-hydroxy-17a$\alpha$-propinyl-D-homo-4,9(10)-16-estratrien-3-on,
11$\beta$-p-Methoxyphenyl-17$\beta$-hydroxy-17$\alpha$-ethinyl-4,9(10)-estradien-3-on oder
11$\beta$-(4-Dimethylaminophenyl)-17$\beta$-hydroxy-17$\beta$-(3-hydroxypropyl)-13$\alpha$-methyl-4,9-gonadien-3-on

4. Verfahren nach Anspruch 1, dadurch gekennzeichnet, daß als langkettige polyungesättigte Fettsäuren solche verwendet werden, die 3 bis 5 isoliert angeordnete Doppelbindungen und 18 bis 22 geradkettig angeordnete C-Atome welche an einem oder 2 C-Atomen der Position 2, 3, 4, 16, 17, 18, 19 oder 20 methyliert oder äthyliert sein können, enthalten, und die Endgruppe -$CO_2X$ besitzen, wobei X Wasserstoff, die Aminogruppe, eine unter sauren Bedingungen abspaltbare Schutzgruppe, ein Metall- oder Aminokation oder die kationische Form eines Ionenaustauschers darstellt.

5. Verfahren nach Anspruch 1, dadurch gekennzeichnet, daß die Fettsäuren 18:2ω-6, 20:4ω-6, 22:5ω-6, 18:3ω-3, 20:5ω-3, 22:6ω-3, 18:3ω-6, 20:3ω-6, 22:3ω-6, 22:4ω-6, 22:4ω-3 verwendet werden.

6. Verfahren nach Anspruch 1, dadurch gekennzeichnet, daß die Fettsäuren 20:4ω-6 und/oder 20:3ω-6 verwendet werden.

7. Verfahren nach Anspruch 1, dadurch gekennzeichnet, daß gegebenenfalls Prostaglandine der E und/oder F-Reihe verwendet werden.

8. Verfahren nach Anspruch 1, dadurch gekennzeichnet, daß Antigestagene und polyungesättigte Fettsäuren in einem Gewichtsverhältnis von 1:1 bis 1:300 verwendet werden.

9. Verfahren nach Anspruch 1, dadurch gekennzeichnet, daß man Antigestagene und polyungesättigte Fettsäuren in einem Gewichtsverhältnis von <1 : 20 verwendet.

10. Verfahren nach Anspruch 1, dadurch gekennzeichnet, daß pro Dosierungseinheit 1 - 500 mg einer polyungesättigten Fettsäure verwendet werden.

11. Verwendung von Antigestagenen und langkettigen polyungsgesättigten Fettsäuren gemäß Anspruch 1, zur gemeinsamen oder gleichzeitigen getrennten oder zeitlich abgestuften Anwendung zur Geburtseinleitung, für den Schwangerschaftsabbruch, oder als Kontrazeptivum.